# EUROPÄISCHE PATENTSCHRIFT

(11) **EP 0 640 331 B1**
(45) Veröffentlichungstag und Bekanntmachung des Hinweises auf die Patenterteilung: **20.10.1999**
(21) Anmeldenummer: 94112557.7
(22) Anmeldetag: 11.08.1994
(51) Int. Cl.: A61F 13/58

(54) **Verschlussvorrichtung für Hygieneartikel, insbesondere Windelverschluss**
Closure device for hygienic articles, particularly diaper closure
Dispositif de fermeture pour produit hygiénique, notamment pour fermeture de couche

(30) Priorität: 17.08.1993 DE 4327589
(43) Veröffentlichungstag der Anmeldung: 01.03.1995
(73) Patentinhaber: THE PROCTER & GAMBLE COMPANY, Cincinnati, Ohio 45202 (US)
(72) Erfinder: Zimmermann, Volker, D-91301 Forchheim (DE); Zimmer, Dagmar, D-91161 Hiltpolstein (DE)
(74) Vertreter: Rau, Manfred, Dr. Dipl.-Ing.

(56) Entgegenhaltungen:
- WO-A-81/03601
- DE-A- 4 202 704
- FR-A- 2 137 855
- FR-A- 2 403 036
- US-A- 4 522 853
- US-A- 4 834 820

## Beschreibung

Die Erfindung betrifft eine Verschlußvorrichtung für Hygieneartikel, wie Inkontinenz-, Kinderhöschenwindeln, Inkontinenzeinlagen oder dergleichen, zur Verbindung zweier Teile des Hygieneartikels, mit den im Oberbegriff des Patentanspruches 1 angegebenen Merkmalen. Insbesondere bezieht sich die Erfindung auf einen Winderverschluß zur Verbindung eines Vorder- und Hinterteils einer Windel im Bereich der Hüftaußenseiten.

Beim Tragen der eingangs erwähnten Hygieneartikel und insbesondere bei Inkontinenzeinlagen oder Windeln für Erwachsene besteht grundsätzlich das Problem, daß eine Anpassung des Artikels an unterschiedliche Körpermaße der Träger möglich sein soll, um angenehme Trageeigenschaften und eine möglichst genaue Paßform zu erzielen. Darüber hinaus sollen die Hygieneartikel beim Tragen zumindest in einem gewissen Maße elastisch nachgiebig sein, um sich an Bewegungen des Trägers anpassen zu können.

Zur Lösung der vorstehenden Probleme wurde bei Inkontinenzeinlagen für Erwachsene bereits vorgeschlagen, als Verschlußvorrichtung zwischen dem Vorder- und Hinterteil der Einlage ein elastisches Band zu verwenden. Als Verbindungselement zur reversibel lösbaren Verbindung des Bandes mit der Inkontinenzeinlage werden dabei Knöpfe an dem Band verwendet, die in entsprechende Knopflöcher an den Einlagen einzuknöpfen sind.

Ein derartiges Verbindungssystem nach dem Stand der Technik ist vergleichsweise aufwendig herzustellen, was mit dem Wegwerf-Charakter solcher Einlagen nur schwer vereinbar ist. Darüber hinaus ist das Anknöpfen des elastischen Bandes relativ mühselig.

Weiterhin ist es insbesondere auf dem Gebiet Von Kinder-Höschenwindeln bekannt, den Windelverschluß durch nicht-dehnbare Klebebänder zu realisieren. Die notwendige Elastifizierung der Höschenwindel zur Erzielung einer befriedigenden Paßform wird dabei durch in die Windel eingearbeitete Elastikteile wie dehnbare Hüftbündchen bewerkstelligt. Auch dies ist konstruktiv, und herstellungstechnisch relativ aufwendig.

Aus der FR-A-2 137 855 ist eine Windel für den einmaligen Gebrauch bekannt, bei der als Verschlußvorrichtung ein halbelastischer Befestigungsstreifen mit einem frei dehnbaren elastischen Mittelabschnitt und zwei nicht dehnbaren unelastischen Endabschnitten vorgesehen ist. Einer dieser Endabschnitte wird auf einem Seitenrand der Windel an deren rückwärtigem Teil befestigt. Der andere Endabschnitt steht von der Windel frei ab und ist mit einem druckempfindlichen Klebebelag versehen. Zur Versteifung der nicht elastischen Endabschnitte wird das elastische Streifenmaterial an seinen Enden mit einem nicht dehnbaren unelastischen Material 12 auf einer Seite beschichtet oder überzogen oder das elastische Material selektiv verhärtet. Wird nun zur Applikation dieser Verschlußvorrichtung auf einen der unelastischen Endabschnitte ein Selbstklebestreifen aufgebracht, so besteht das Problem, daß dieser für eine Zwischenlagerung der Verschlußvorrichtung auf einer geeigneten Fläche wiederablösbar appliziert oder mit einem Schutzpapier überzogen sein muß. In dieser Hinsicht ist die in FR-A-2 137 855 gezeigte Ausbildung einer Windelverschlußvorrichtung nicht ausgelegt. Dieser Stand der Technik bildet den Oberbegriff des Anspruchs 1.

Die WO-A-8103601 zeigt elastische Verschlußbänder für Höschenwindeln, bei der ein elastischer Bandabschnitt an seinen beiden Enden mit einem darunterliegenden über die beiden Enden hinaus verlängerten unelastischen Bandabschnitt verbunden ist. Letztere ist durch eine Perforation auftrennbar, so daß die beiden unelastischen Endabschnitte unter Dehnung des elastischen Zwischenabschnittes auf das vordere bzw. rückwärtige Teil einer Windel appliziert werden können.

Ausgehend vom den geschilderten Problemen beim Stande der Technik liegt der Erfindung die Aufgabe zugrunde, eine Verschlußvorrichtung für Hygieneartikel der gattungsgemäßen Art so zu verbessern, daß unter Beibehaltung der elastischen Eigenschaften der Verschlußvorrichtung deren Verbindungselement konstruktiv vereinfacht und bequemer handhabbar ist.

Diese Aufgabe wird durch die im Anspruch 1 angegebenen Merkmale gelöst. Demnach weist das Verbindungselement in an sich bekannter Weise am elastischen Band eine das Band zumindest in einem Teilbereich versteifende, unelastisch machende Auflage auf, an der ein Selbstklebeelement zur reversibel lösbaren Verbindung des Bandes mit dem Artikel angebracht ist. Durch die Versteifung des Bandes in einem Teilbereich wird dabei die Grundlage dafür geschaffen, daß ein Selbstklebelement in Verbindung mit einem ansonsten elastischen Band verwendet werden kann. Somit weist die Verschlußvorrichtung einerseits die geforderten elastischen Eigenschaften auf, andererseits können aufwendige Verbindungselemente, wie die Knöpfe und Knopflöcher bei der Verschlußvorrichtung gemäß dem Stand der Technik völlig entfallen.

Durch die Herumführung der versteifenden Auflage um des Bandende von dessen einer auf die andere Seite kann die versteifende Auflage eine Doppelfunktion erfüllen. Einerseits dient sie zur haltbaren Befestigung eines als Selbstklebestreifen ausgebildeten Selbstklebeelementes, andererseits kann die entsprechend beidseitige Auflage nach Art eines sogenannten Release-Tapes wirken, wie es bei herkömmlichen Windel-Klebeverschlüssen verwendet wird. Dabei wird der Selbstklebestreifen mit seiner gesamten Klebefläche zur Zwischenlagerung der Verschlußvorrichtung auf die Auflage aufgeklebt und ist somit vor Verschmutzung und dergleichen geschützt.

Da das Selbstklebelement ferner aus einem Selbstklebestreifen besteht, der naturgemäß flexibel ist, kann sich der auf den Hygieneartikel aufzuklebende Teil der Verschlußvorrichtung flexibel an die Körperform des Trägers anpassen und ruft damit keinerlei Druckstellen od.dgl. hervor. Darüber hinaus ergibt sich durch diese Flexibilität auch ein optimiertes Klebeverhalten.

Grundsätzlich kann die erfindungsgemäße Verschlußvorrichtung mit einem Ende des elastischen Bandes dauerhaft, z.B. am Hinterteil einer Windel oder Inkontinenzeinlage, befestigt und mit dem anderen Bandende reversibel lösbar, z.B. mit dem Vorderteil der Windel oder Einlage verbunden werden. In diesem Falle bildet die Verschlußvorrichtung eine Einheit mit der Windel und wird mit ihr weggeworfen.

Andererseits kann eine erfindungsgemäße Verschlußvorrichtung auch als wiederverwendbares Teil zusammen mit Wegwerf-Windeln oder -Einlagen verwendet werden. Dazu ist gemäß Anspruch 2 vorgesehen, daß jeweils eine das elastische Band versteifende Auflage in den beiden Endbereichen des Bandes angeordnet ist. Entsprechend kann jeweils ein Selbstklebeelement zur reversibel lösbaren Verbindung des Bandes mit dem Artikel an den beiden Bandenden befestigt werden. Die erfindungsgemäße Verschlußvorrichtung bildet also ein wiederverwendbares Verschlußsystem, das vielfach mit z.B. Wegwerf-Erwachsenenwindeln verwendet werden kann. Dies bedingt eine weitere Kostensenkung bei der Herstellung und Anwendung der erfindungsgemäßen Verschlußvorrichtung bzw. der entsprechenden Hygieneartikel, da nach Gebrauch der Artikel selbst entsorgt wird, die Verschlußvorrichtung aber weiter einsetzbar ist.

Die Ansprüche 3 bis 5 kennzeichnen vorteilhafte Ausführungsformen für die versteifenden Auflagen an dem elastischen Band. Dabei können z.B. Kunststoff-Folienstreifen verwendet werden, die aus Polyester bestehen. Auch imprägnierte oder silikonisierte Papierstreifen sind denkbar.

Die Ansprüche 6 bis 8 kennzeichnen vorteilhafte Ausbildungen des Selbstklebeelementes, das zur jeweiligen Verbindung der unelastischen Auflage des elastischen Bandes mit der Windel dient.

Nach Anspruch 9 kann das elastische Band der Verschlußvorrichtung aus einem z.B. textilen Gummiband, einem elastischen Vlies, einem elastischen Gittergeflecht oder einer elastischen Folie bestehen, die nur in der Längsrichtung des Bandes elastisch sein muß.

Um die erfindungsgemäße Verschlußvorrichtung weiter zu optimieren, ist nach Anspruch 10 vorgesehen, daß am jeweiligen Bandende sowohl auf der Innen- als auch auf der Außenseite versteifende Auflagen mit unterschiedlichen Haltstärken für einen darauf anzubringenden Selbstklebestreifen angebracht sind. So läßt sich z.B. durch eine silikonisierte Polyester-Folie oder ein silikonisiertes Papier auf der einen Seite eine relativ geringe Haftkraft für einen darauf anzubringenden Selbstklebestreifen erzielen. Eine Auflage mit einer solchen geringen Haftkraft wird dann dort eingesetzt, wo die Auflage als Release-Tape fungiert, wo also der Selbstklebestreifen mit seiner Klebefläche nur zur Zwischenlagerung bis zur nächsten Wiederverwendung aufgeklebt wird.

Derselbe Effekt kann im übrigen dadurch erzielt werden, daß die sich nach Anspruch 1 um das Bandende herum erstreckende Auflage auf der einen Seite noch mit einer entsprechenden Beschichtung - beispielsweise einer Silikonisierung - versehen ist.

Weitere Merkmale, Einzelheiten und Vorteile der Erfindung sind der nachfolgenden Beschreibung entnehmbar, in der Ausführungsbeispiele des Erfindungsgegenstandes anhand der beiliegenden Zeichnungen näher erläuwerden. Es zeigen:
- Fig. 1: eine perspektivische Darstellung einer Inkontinenz-Windel für Erwachsene mit einer erfindungsgemäßen Verschlußvorrichtung in Schließstellung,
- Fig. 2: einen schematischen Horizontal-Längsschnitt durch eine Verschlußvorrichtung der Windel entlang der Schnittlinie II-II nach Fig. 1,
- Fig. 3: einen schematischen Horizontal-Längsschnitt einer Verschlußvorrichtung in stark vergrößertem Maßstab,
- Fig. 4: einen schematischen Horizontal-Längsschnitt durch eine erfindungsgemäße Verschlußvorrichtung, welcher Längsschnitt eine zweite und dritte Ausführungsform zeigt, und
- Fig. 5: einen teilweisen, schematischen Horizontal-Längsschnitt einer vierten Ausführungsform der Erfindung.

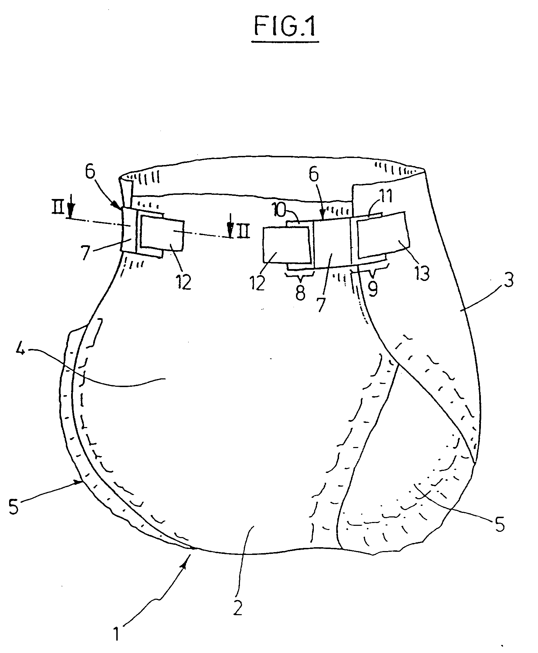

Die in Fig. 1 dargestellte Inkontinenzwindel 1 weist einen üblichen, nicht näher dargestellten Windelaufbau mit einem Zellstoff-Flocken-Saugkörper, einer körperseitigen Vliesstoffauflage und einer dem Körper abgewandten, flüssigkeitsundurchlässigen Abdecklage 2 aus dünner Polyethylen-Folie auf. Das Hinterteil 3 und das Vorderteil 4 der Inkontinenzwindel 1 überlappen einander im angelegten Zustand der Windel und bilden dabei Beinöffnungen 5.

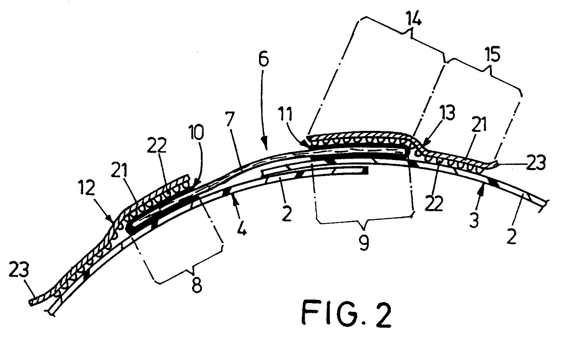

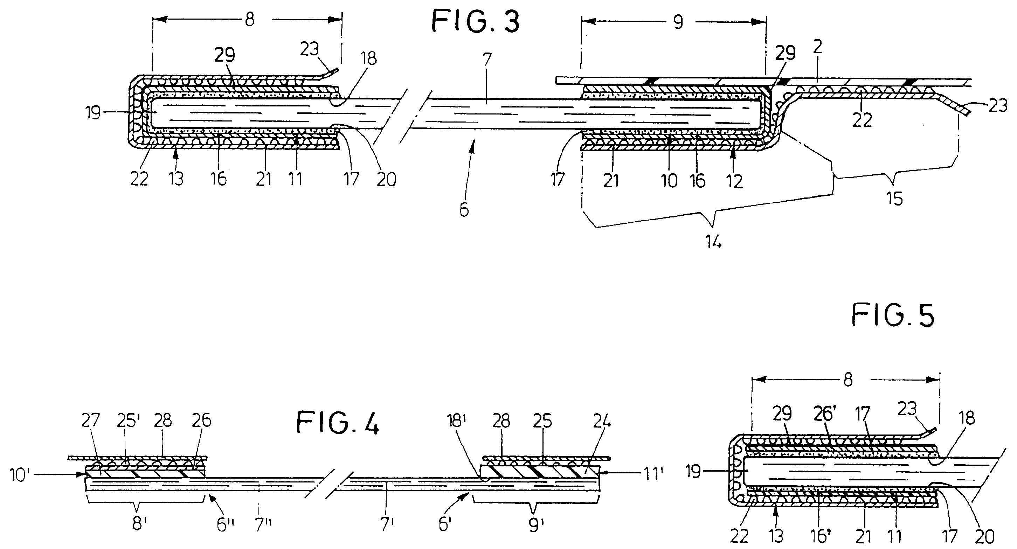

Vorder- 4 und Hinterteil 3 sind durch Verschlußvorrichtungen 6 im Bereich der Hüftaußenseiten miteinander verbunden. Diese Verschlußvorrichtung 6 besteht im wesentlichen aus einem langgestreckten, elastischen Band 7, bei dem es sich um ein textiles Band aus Polyester-Fäden mit eingewebten Gummifäden handelt. Wie aus den Fig. 2 und 3 deutlicher hervorgeht weist das elastische Band 7 in seinen beiden Endbereichen 8,9 beidseitig eine versteifende, das elastische Band 7 unelastisch machende Auflage 10,11 auf, die jeweils als Befestigungsbasis für Selbstklebestreifen 12,13 dienen. Letztere sind mit ihrer einen Längshälfte 14 mit der Auflage 10 bzw. 11 verbunden und mit der zweiten Längshälfte 15 im angelegten Zustand mit der Abdecklage 2 der Windel 1 verklebt.

Aufgrund der vorstehend geschilderten Ausgestaltung der Verschlußvorrichtung 6 wird einerseits eine zuverlässige Fixierung der beiden Windelteile 3,4 zueinander erzielt. Andererseits kann sich die Windel 1 durch die Elastizität des Bandes 7 zwischen den beiden Endbereichen 8,9 optimal an den Träger des Körpers anpassen und auch bei Bewegungen des Trägers einen einwandfreien Sitz der Windel gewährleisten.

Eine bevorzugte Ausführungsform einer erfindungsgemäßem Verschlußvorrichtung 6 ist anhand von Fig. 3 näher zu erläutern. Hierbei bestehen die beiden Auflagen 10,11 in den beiden Endbereichen 8,9 des Bandes 7 jeweils aus einem rechteckigen Kunststoff-Folienstreifen 16 aus einer nicht-dehnbaren Polyesterfolie mit einer Dicke von 125 µm. Die Streifen 16 sind jeweils mittels einer Klebeschicht 17 aus einem wärmeaktivierbaren Polyester-Schmelzkleber in den Endbereichen 8,9 des Bandes 7 mit diesem verklebt. Dabei werden die Streifen 16 so aufgebracht, daß sie sich von einer Längsseite 18 des elastischen Bandes 7 um das Bandende 19 herum auf die zweite Längsseite 20 erstrecken. Wie durch eine verdickte Linie in Fig. 3 angedeutet ist, weist der Kunststoff-Folienstreifen 16 auf der im angelegten Zustand der Windel zugekehrten Seite - also auf der der Längsseite 18 des elastischen Bandes 7 zugewandten Seite - eine Beschichtung 29 in Form einer Silikonisierung auf. Durch diese Beschichtung 29 haften die Selbstklebestreifen 12, 13 in diesen Bereich weniger als auf dem unbeschichteten Teil der Kunststoff-Folienstreifen 16. Dadurch können die Selbstklebestreifen 12, 13 leichter von dieser Seite der Auflagen 10, 11 abgezogen werden. Damit ist die Handhabung des erfindungsgemäßen Windel-Verschlußsystems weiter vereinfacht.

Bei der Anbringung der Auflagen 10,11 wird dabei so verfahren, daß eine Bahn der Polyesterfolie mittels eines Rotationsschmelzsiebdruck-Verfahrens mit der Klebeschicht 17 versehen wird. Dabei kommt ein Sieb mit einer Maschenweite von 11 mesh zum Einsatz, mit dem der Klebstoff mit einem Auftragsgewicht von 50 g/m² unter Bildung kleiner Noppen aufgebracht wird. Die derart beschichtete Bahn wird zu Streifen 16 geschnitten, die wie in Fig. 3 dargestellt, in ungedehntem Zustand des Bandes 7 um dessen Endbereiche 8,9 herumgelegt werden. Anschließend werden die Streifen 16 durch Pressen und Temperatureinwirkung beidseitig aufgebügelt, so daß ein fester Verbund der Streifen 16 mit dem Band 7 in den Endbereichen 8,9 entsteht. Durch diesen festen Verbund ist das elastische Band in den Endbereichen 8,9 nicht mehr dehnbar. Damit können die Auflagen 10, 11 als stabiler Untergrund für die Anbringung eines Klebeelements zur Verbindung des Bandes 7 mit der Windel 1 dienen.

Die anhand Fig. 1 allgemein angesprochenen Selbstklebestreifen 12,13 bestehen aus einem streifenförmigen imprägnierten Papierträger 21, der auf einer Seite mit einer Beschichtung 22 aus einer selbstklebenden Heißschmelzkleber-Masse auf der Basis von Styrol-Isopren-Styrol-Blockcopolymeren versehen ist. Die Beschichtung 22 ist mit Hilfe eines Rotationsschmelzsiebdruckverfahrens mittels eines 25 mesh-Siebes und mit einem Auftragsgewicht von 35 g/m² aufgebracht.

Wie aus Fig. 3 deutlich wird, können die Selbstklebestreifen 12,13 in einem Lagerzustand mit ihrer vollen Länge auf den Auflagen 10,11 aufgeklebt sein (siehe Fig. 3, linker Teil), wodurch letztere als Release-Tape wirken.

Bei Verwendung der Verschlußvorrichtung 6 beim Anlegen der Windel 1 werden die Selbstklebestreifen 12,13 zur Hälfte abgezogen, was durch das von der Beschichtung 22 freibleibende Griffende 23 an den Klebestreifen 12,13 erleichtert wird. Die von der Auflage 10,11 abgezogene Längshälfte 15 wird anschließend auf die Abdecklage 2 der Windel 1 aufgedrückt und somit verklebt (s. Fig. 2 sowie Fig. 3, rechter Teil). Diese Verklebung ist aufgrund der Beschaffenheit und noppenförmigen Ausgestaltung der Beschichtung 22 reversibel lösbar, so daß der Selbstklebestreifen 12,13 von einer verbrauchten Windel 1 abgezogen und die gesamte Verschlußvorrichtung 6 bei einer neuen Windel wiederverwendbar ist. Bis zur Wiederverwendung können die Selbstklebestreifen 12,13 auf den Auflagen 10,11, wie in Fig. 3, linker Teil dargestellt, zwischengelagert werden. Weiterhin können nach einer vielfachen Benutzung der Selbstklebestreifen 12,13 und einem entsprechenden Nachlassen der Klebekraft die Selbstklebestreifen 12, 13 im Bereich ihrer Längshälften 14 von den Auflagen 10,11 komplett abgezogen und neue Klebestreifen aufgebracht werden.

Im übrigen wird zum Herstellungsverfahren der Beschichtung 22 bzw. der Klebeschicht 17 auf die deutsche Patentanmeldung P 42 02 704.7 verwiesen, in der das verwendete Rotationsschmelzsiebdruckverfahren eingehend erörtert ist.

Bei dem in Fig. 4, rechter Teil gezeigten Ausführungsbeispiel einer Verschlußvorrichtung 6' ist wiederum ein elastisches Band 7' vorhanden, das in seinen beiden Endbereichen 9' auf einer Seite mit einer Auflage 11' versehen ist, die aus einer thermoplastischen Beschichtung 24 auf einer Längsseite 18' des Bandes besteht. Die Beschichtung 24 wird beispielsweise durch Angießen eines geschmolzenen thermoplastischen Kunststoffes hergestellt, der sich innig mit der Textilstruktur des elastischen Bandes 7' verbindet und nach dem Erkalten fest mit dem elastischen Band 7' verankert ist. Auf ihrer Oberseite ist die Beschichtung 24 wiederum mit einer aus kleinen Noppen gebildeten Klebeschicht 25 versehen, die mit Hilfe des bereits erwähnten Rotationsschmelzsiebdruckverfahrens aufgebracht wird. Damit kann die Verschlußvorrichtung 6' wiederum reversibel lösbar mit einer Windel 1 verbunden werden.

In Fig. 4, linker Teil ist ein Ausführungsbeispiel einer Verschlußvorrichtung 6'' gezeigt, bei dem die grundsätzliche Machart der das Gummiband 7'' unelastisch machenden Auflage 10' dem Ausführungsbeispiel gemäß Fig. 3 entspricht. Dabei ist ein Stanzling auf der Basis eines imprägnierten Papierstreifens 26 vorgesehen, der auf seiner Außenseite mit der bereits erwähnten noppenförmigen Klebeschicht 25' aus selbstklebendem Klebstoff versehen ist. Diese Klebeschicht 25' ist wiederum mit dem bereits erwähnte Rotationsschmelzsiebdruckverfahren aufzubringen. Auf der Innenseite des Papierstreifens 26 ist eine permanent selbstklebende Klebeschicht 27 vorgesehen, mit der der Papierstreifen 26 dauerhaft mit dem elastischen Band 7'' verbunden wird. Durch diese feste Verbindung ist der Endbereich 8' wiederum unelastisch gemacht. Die Innenseite des Papierstreifens 26 kann im übrigen auch mit einer wärmeaktivierbaren Klebeschicht beschichtet werden, wobei durch ein Schmelzen des Klebers die permanente Verbindung hergestellt wird.

Bei beiden Ausführungsformen gemäß Fig. 4 ist auf der Klebeschicht 25, 25' ein Abdeckstreifen 28 aus silikonisiertem Papier aufgebracht, der vor Einsatz der Verschlußvorrichtung 6' bzw. 6'' zum Freilegen der Klebeschicht 25, 25'' abgezogen wird.

Das in Fig. 5 gezeigte Ausführungsbeispiel entspricht im wesentlichen dem Ausführungsbeispiel gemäß Fig. 3. Übereinstimmende Teile sind daher mit übereinstimmenden Bezugszeichen versehen. Als Unterschied ist jedoch der Kunststoff-Folienstreifen 16' nicht um das Bandende 19 herumgezogen, sondern nur im Bereich der außenseitigen Längsseite 20 angebracht. Es handelt sich dabei um einen unbeschichteten Polyester-Folienstreifen, auf dem der Selbstklebestreifen 13 sehr gut haftet. Auf der innenseitigen Längsseite 18 ist ein Papierstreifen 26' über die Klebeschicht 17 aufgebracht, der auf seiner Außenseite wiederum eine Beschichtung 29 in Form einer Silikonisierung aufweist. Damit läßt sich der Selbstklebestreifen 13 leichtgängig aus der in Fig. 5 gezeigten "Zwischenlagerstellung" von dem Papierstreifen 26' ablösen und in die in Fig. 3, rechter Teil gezeigte Aktivstellung verbringen.

## Patentansprüche

1. Verschlußvorrichtung für Hygieneartikel, wie Inkontinenz-, Kinderhöschenwindeln, Inkontinenzeinlagen od.dgl., zur Verbindung zweier Teile des Hygieneartikels, insbesondere Windelverschluß zur Verbindung eines Vorder- (4) und Hinterteils (3) einer Windel (1) im Bereich der Hüftaußenseiten, mit
- einem elastischen Band (7,7') zwischen den zu verbindenden Teilen (4,3) des Artikels (1) und
- mit mindestens einem Verbindungselement zur reversibel lösbaren Verbindung des Bandes (7,7') mit mindestens einem der beiden zu verbindenden Teile (4,3) des Artikels (1), wobei das mindestens eine Verbindungselement eine das elastische Band (7,7') in einem Teilbereich versteilende, unelastisch machende Auflage (10,11, 10',11') aufweist, an der ein Selbstklebeelement (12,13) Klebeschicht 25) zur reversibel lösbaren Verbindung des Bandes (7) mit dem Artikel (1) angebracht ist, dadurch gekennzeichnet, daß sich die jeweilige Auflage (10, 11) von einer Seite (18) des elastischen Bandes (7) auf dessen zweite Seite (20) um das Bandende (19) herum erstreckt, und daß das Selbstklebeelement aus einem Selbstklebestreifen (12, 13) besteht, der einerseits mit der versteifenden Auflage (10, 11) verbunden und andererseits reversibel lösbar mit dem Artikel (1) verbindbar ist.

2. Verschlußvorrichtung nach Anspruch 1, dadurch gekennzeichnet, daß jeweils eine Auflage (10,11,10'11') in den beiden Endbereichen (8,9, 8',9') des elastischen Bandes (7,7') angeordnet ist, so daß die Verschlußvorrichtung als wiederverwendbares Verschlußsystem vielfach mit Wegwerf-Hygieneartikeln reversibel lösbar zu verbinden ist.

3. Verschlußvorrichtung nach Anspruch 1 oder 2, dadurch gekennzeichnet, daß die versteifende Auflage (10,11,10') aus einem mit dem Band (7,7'') verklebten, nicht-dehnbaren Kunststoff-Folienstreifen (16, 16') oder Papierstreifen (26, 26') besteht.

4. Verschlußvorrichtung nach Anspruch 3, dadurch gekennzeichnet, daß der Kunststoff-Folienstreifen (16, 16') oder Papierstreifen (26, 26') mittels eines wärmeaktivierbaren Schmelzklebers (Klebeschicht 17) oder einer selbstklebenden Klebeschicht (27) mit dem Band (7,7'') dauerhaft verliebt ist.

5. Verschlußvorrichtung nach Anspruch 1 oder 2, dadurch gekennzeichnet, daß die Auflage (11') aus einer kalt aushärtbaren oder thermoplastischen Beschichtung (24) besteht.

6. Verschlußvorrichtung nach einem der Ansprüche 1 bis 5, dadurch gekennzeichnet, daß das Selbstklebeelement aus einer direkt auf die jeweilige Auflage (10', 11') aufgebrachten Klebeschicht (25, 25') gebildet ist.

7. Verschlußvorrichtung einem der nach Ansprüche 1 bis 6, dadurch gekennzeichnet, daß der Selbstklebestreifen (12, 13) reversibel lösbar mit der versteifenden Auflage (10, 11) verbunden ist.

8. Verschlußvorrichtung nach Anspruch 1, 4 und/oder 7, dadurch gekennzeichnet, daß die Klebeschicht (17) zur Verbindung des Kunststoff-Folienstreifens (16, 16') oder Papierstreifen (26, 26') mit dem Band (7, 7''), die Beschichtung (22) des Selbstklebestreifens (12, 13) und/oder die Klebeschicht (25, 25') jeweils aus Schmelzklebstoffen bestehen, die durch ein Rotationsschmelzsiebdruckverfahren aufgebracht sind.

9. Verschlußvorrichtung nach einem der Ansprüche 1 bis 8, dadurch gekennzeichnet, daß das elastische Band (7, 7', 7'') aus einem Gummiband, einem elastischen Vlies, einem elastischen Gittergeflecht oder einer elastischer Folie besteht.

10. Verschlußvorrichtung nach einem der Ansprüche 1 bis 9 dadurch gekennzeichnet, daß am jeweiligen Bandende (19) sowohl auf der einen Längsseite (18), als auch auf der zweiten Längsseite (20) versteifende Auflagen (Kunststoff-Folienstreifen 16', Papierstreifen 26') aufgebracht sind, die auf diesen beiden Seiten unterschiedlich ausgebildet sind, um unterschiedliche Haftstärken für den darauf anzubringenden Selbstklebestreifen (13) zu erzielen.

## Claims

1. A closure device for hygienic articles, such as incontinent briefs, children's diaper pants, incontinent pads or the like, for joining to each other two portions of the hygienic article, in particular a diaper closure for joining the front portion (4) to the rear portion (3) of a diaper (1) in the vicinity of the lateral sides of the hips, comprising
- an elastic band (7, 7') between the portions (4, 3), to be joined, of the article (1); and
- at least one connecting element for the reversibly releasable connection of the band (7, 7') with at least one of the two portions (4, 3), to be joined, of the article (1),
the at least one connecting element comprising a layer (10, 11, 10', 11') which rigidifies a partial area of the elastic band (7, 7'), making it inelastic, and on which a self-adhesive element (12, 13, adhesive layer 25) is mounted for the reversibly releasable connection of the band (7) with the article (1), characterized in that the respective layer (10, 11) extends from one side (18) of the elastic band (7) onto the second side (20) thereof around the end (19) of the band (7), and in that the self-adhesive element is a self-adhesive tape (12, 13) joined to the rigidifying layer (10, 11) on the one hand and to be reversibly releasably connected with the article (1) on the other.

2. A closure device according to claim 1, characterized in that a layer (10, 11, 10', 11') is disposed in the two end portions (8, 9, 8', 9') of the elastic band (7, 7') so that repeated reversibly releasable connection of the closure device as a re-usable fastening system with disposable hygienic articles is possible.

3. A closure device according to claim 1 or 2, characterized in that the rigidifying layer (10, 11, 10') consists of a tape of plastic film (16, 16') or a paper tape (26, 26') which are non-extensible and glued on the band (7, 7').

4. A closure device according to claim 3, characterized in that the tape of plastic film (16, 16') or the paper tape (26, 26') is durably glued on the band (7, 7'') by means of a thermally activable hot-melt-type adhesive (adhesive layer 17) or a self-adhesive layer (27).

5. A closure device according to claim 1 or 2, characterized in that the layer (11') consists of a cold-curing or thermoplastic coating (24).

6. A closure device according to one of claims 1 to 5, characterized in that the self-adhesive element is formed by an adhesive layer (25, 25') directly applied on the respective layer (10', 11').

7. A closure device according to one of claims 1 to 6, characterized in that the self-adhesive tape (12, 13) is reversibly releasably joined to the rigidifying layer (10, 11).

8. A closure device according to claim 1, 4 and/or 7, characterized in that the adhesive layer (17) for joining the tape of plastic film (16, 16') or the paper tape (26, 26') to the band (7, 7''), the coating (22) of the self-adhesive tape (12, 13) and/or the adhesive layer (25, 25') each consist of hot-melt-type adhesives which are applied by rotational casting serigraphy.

9. A closure device according to one of claims 1 to 8, characterized in that the elastic band (7, 7', 7'') consists of a rubber tape, a elastic nonwoven, an elastic lattice structure or an elastic film.

10. A closure device according to one of claims 1 to 9, characterized in that rigidifying layers (tapes of plastic film 16', paper tapes 26') are applied to the respective end (19) on the first lengthwise side (18) as well as on the second lengthwise side (20), these rigidifying layers (tapes of plastic film 16', paper tapes 26') being designed differently on these two sides so that varying adhesive strengths are obtained for the self-adhesive tape (13) to be applied thereto.

## Revendications

1. Dispositif de fermeture pour articles hygiéniques tels que des couches d'incontinence, des couches culottes de bébé, des garnitures d'incontinence ou analogues, destiné à la liaison de deux parties de l'article hygiénique, en particulier fermeture de couche destinée à la liaison d'une partie avant (4) et d'une partie arrière (3) d'une couche (1) au niveau des côtés extérieurs des hanches, comportant
- une bande élastique (7, 7') entre les parties (4, 3) de l'article (1), et
- au moins un élément de liaison destiné à la liaison amovible réversible de la bande (7, 7') avec l'une au moins des deux parties (4, 3) à relier de l'article (1), le seul élément de liaison au moins comportant un revêtement (10, 11, 10', 11') renforçant la bande élastique (7, 7') dans une zone partielle et la rendant non élastique, sur lequel est fixé un élément autoadhésif (12, 13) (couche de colle 25) destiné à la liaison amovible réversible de la bande (7) avec l'article (1), caractérisé en ce que chaque revêtement (10, 11) s'étend autour de l'extrémité (19) de la bande sur une face (18) de la bande élastique (7) vers sa deuxième face (20), et en ce que l'élément auto-adhésif est constitué d'un ruban auto-adhésif (12, 13), qui est d'une part relié au revêtement de renfort (10, 11), et qui peut d'autre part être relié de façon amovible réversible à l'article (1).

2. Dispositif de fermeture selon la revendication 1, caractérisé en ce qu'un revêtement (10, 11, 10', 11') est à chaque fois disposé dans les deux zones d'extrémité (8, 9, 8', 9') de la bande élastique (7, 7'), de sorte que le dispositif de fermeture peut être relié de multiples fois à des articles hygiéniques jetables en tant que système de fermeture réutilisable.

3. Dispositif de fermeture selon la revendication 1 ou 2, caractérisé en ce que le revêtement de renfort (10, 11, 10') est constitué d'un ruban de film (16, 16') non extensible en matière plastique ou d'une bande de papier (26, 26') collé sur la bande (7, 7'').

4. Dispositif de fermeture selon la revendication 3, caractérisé en ce que le ruban de film (16, 16') en matière plastique ou la bande de papier (26, 26') est collé de façon durable sur la bande (7, 7'') au moyen d'une colle fusible thermo-activable (couche de colle 17), ou d'une couche de colle (27) auto-adhésive.

5. Dispositif de fermeture selon la revendication 1 ou 2, caractérisé en ce que le revêtement (11') est constitué d'un revêtement (24) durcissable à froid ou thermoplastique.

6. Dispositif de fermeture selon l'une des revendications 1 à 5, caractérisé en ce que l'élément auto-adhésif est constitué d'une couche de colle (25, 25') appliquée directement sur le revêtement respectif (10', 11').

7. Dispositif de fermeture selon l'une des revendications 1 à 6, caractérisé en ce que le ruban auto-adhésif (12, 13) est relié de façon amovible réversible au revêtement de renfort (10, 11).

8. Dispositif de fermeture selon la revendication 1, 4 et/ou 7, caractérisé en ce que la couche de colle (17) destinée à relier le ruban de film (16, 16') en matière plastique ou la bande de papier (26, 26') à la bande (7, 7''), le revêtement (22) du ruban auto-adhésif (12, 13) et/ou la couche de colle (25, 25'), sont respectivement constitués de colles fusibles qui sont appliquées par un procédé de sérigraphie par fusion et rotation.

9. Dispositif de fermeture selon l'une des revendications 1 à 8, caractérisé en ce que la bande élastique (7, 7', 7'') est constituée d'une bande de caoutchouc, d'un non-tissé élastique, d'un réseau tissé élastique ou d'un film élastique.

10. Dispositif de fermeture selon l'une des revendications 1 à 9, caractérisé en ce que des revêtements de renfort (rubans de film 16' en matière plastique, bandes de papier 26') sont à chaque fois appliqués sur l'extrémité (19) de la bande, aussi bien sur l'une de ses faces longitudinales (18) que sur la deuxième face longitudinale (20), qui sont réalisés différemment sur ces deux faces afin d'obtenir différentes forces d'adhérence pour le ruban auto-adhésif (13) devant y être appliqué.
